# EUROPEAN PATENT SPECIFICATION

(11) **EP 2 885 449 B1**
(45) Date of publication and mention of the grant of the patent: **01.11.2017**
(21) Application number: 12756278.3
(22) Date of filing: 17.08.2012
(51) Int. Cl.: D01F 6/92, D04H 1/435, D04H 1/55, D06B 1/14, A61L 17/12, C08G 63/08, D01F 6/84, D01F 1/10, A61L 15/26, D04B 1/14

(54) **BIODEGRADABLE TEXTILES AND METHODS OF THEIR MANUFACTURE**
BIOLOGISCH ABBAUBARE TEXTILIEN UND VERFAHREN ZU IHRER HERSTELLUNG
TEXTILES BIODÉGRADABLES ET LEUR PROCÉDÉ DE FABRICATION

(43) Date of publication of application: 24.06.2015
(73) Proprietor: Politechnika Lódzka, 90-924 Lódz (PL); Instytut Biopolimerów I Wlókien Chemicznych, 90-570 Lódz (PL); Centrum Materialow Polimerowych i Weglowych PAN, 41-819 Zabrze (PL); Instytut Wlókiennictwa, 92-103 Lódz (PL)
(72) Inventor: KRUCINSKA, Izabella, 91-857 Lódz (PL); CHRZANOWSKI, Michal, 91-320 Lódz (PL); KOWALSKA, Stanislawa, 91-372 Lódz (PL); KOMISARCZYK, Agnieszka, 91-473 Lódz (PL); TWAROWSKA-SCHMIDT, Krystyna, 93-328 Lódz (PL); CIECHANSKA, Danuta, 93-355 Lódz (PL); SULAK, Konrad, 95-050 Konstantynów L. (PL); OLCZYK, Krzysztof, 93-503 Lódz (PL); DOBRZYNSKI, Piotr, 41-808 Zabrze (PL); KOWALCZUK, Marek, 41-800 Zabrze (PL); PASTUSIAK, Malgorzata, 41-803 Zabrze (PL); SOBOTA, Michal, 42-224 Czestochowa (PL); MIELICKA, Elzbieta, 91-403 Lódz (PL); PINAR, Anna, 93-248 Lódz (PL); ZYWICKA, Boguslawa, 53-152 Wroclaw (PL)
(74) Representative: Dziubinska, Joanna
(86) International application number: PCT/PL2012/000069
(87) International publication number: WO 2014/027906

(56) References cited:
- EP-A1- 0 600 237
- EP-A1- 1 375 706
- EP-A2- 1 038 540
- WO-A1-2009/152349
- US-A- 3 839 297
- US-A- 5 320 624
- US-B1- 6 277 927

## Description

The invention relates to biodegradable textiles and methods of the manufacture of biodegradable textiles primarily for medical uses.

Biodegradable polymeric fibers made of polylactide by melt spinning are described, amongst other, in following publications: monograph "Biodegradable and sustainable fibres," edited by R.S.Blackburn, Woodhead Publishing Limited, 2005, "Biodegradable fibres made of poly-lactic acid" in Chemical Fibres International, vol.46, no.5, 1996r. and monograph "Poly(lactic acid):Synthesis, Structures, Properties and Application" editor .R.Auras L-T, et al, 2010..Thanks to easy processing by melt spinning , unique properties and renewable resources, polylactide fibers have found application in the production of a wide range of textiles like apparel, home textiles, nonwoven for agriculture and hygiene, and geotextiles.

The use of polylactide as at least one of the components of staple- or continuous fibers is known from the American- US 6506873 and European EP 0977912 patents. According to these patents, textiles are prepared from the melts of lactide- polymers and copolymer and their blends with other biodegradable and non-degradable polymers by melt spinning, spun bond and melt blown techniques. Nonwoven described in the patents can have utility in agriculture, and medical-, hygienic- and filtration materials.

Known from the American patents : US 5525706, US 5807973, US 6111060 and US 6355772 are nonwoven products made of compositions of lactide polymers containing additives which stabilize the polymer in the course of processing. The applied polymeric lactide compositions contain polylactide bonds as the greater part, unreacted lactide in the amount of less than 2% and water in an amount below 2000 ppm. The nonwoven is made by direct spinning from the melt (spun bond and melt blown techniques) or by forming from staple fibers. On account of various additives contained in the lactide polymer, the biodegradable textiles as described in above patents are not suited for uses in medical bioresorbable devices.

Known from the US 6905987 and EP1381720 patents are biodegradable melt-spun fibers containing copolymers of poly(hydroxalkanoates) and polymers or copolymers of lactic acid. The fibers may be formed either as mono-component made of a polymer blend or bi-component of the sheath/ core type where the sheath is made up of lactide polymers while the core contains poly(hydroxalkanoates) copolymers.Described are also nonwoven materials prepared with a content of at least 2% of such fibers where the nonwoven is made by direct spinning from the melt (spun bond) or by forming from staple fibers. The nonwoven can have utility in many products : hygienic like diapers, napkins, suspensory, and medical like dressing materials, surgical bandages and dental threads.

Biodegradable polymeric fibers made of aliphatic polyesters and co-polyesters such as polyglycolide and polylactide by melt-spinning and drawing are also known from the US 2006/0159918. The invention relates to continuous and staple fibers ,crimped or smooth, mono- and bi-component. Thanks to special spinning conditions and/or use of crystallization-retarding additives, the fibers maintain their tenacity when stored at ambient temperature of 20-30 °C and relative humidity of 70-100%.

Known is also the use of lactide/glycolide copolymers in the manufacture of medical products inclusive fibers. Multifilament surgery sutures are made of a polymer containing 90% of glycolic acid and 10 % of L-lactic acid. Surgery sutures obtained from a polymer containing a higher amount of lactide than glycolide to slow down the degradation process are known from the publication "Biodegradable polymers as biomaterials", Lakshmi S. Nair, Cato T. Laurencin, Prog.Polym.Sci 32,2007,762-798.

A method for the manufacture of knitwear designed for dressings made of bioresorbable polymers on the base of glycolide, lactide and/or lactones.is known from the Polish patent application P.389377 and from the publication "Assessment of a new generation of biodegradable copolymers for uses in the technology of medical knitwear") in the periodical "Techniczne Wyroby Wlókiennicze", 2008, p.56-62. Notwithstanding the above described solutions for the preparation of fibers and textiles containing lactide or copolymers of lactide with other polymers, effective methods are still sought for the manufacture of lactide products bearing properties adequate to medical application.

The present invention relates to a technology for the production of textiles and its application in the manufacture of desirable biodegradable materials for mainly medical uses.

Biodegradable textiles of the present invention containing a plurality of fibers prepared from the melt of a homogeneous polymer blend having average molecular mass of 100000 - 270000, glass transition temperature of 45 - 47°C, melting temperature of 149 - 152°C and enthalpy of fusion above 30J/g, composed in 82-98% wt. of lactide / glycolide copolymer, and in 2 - 18% wt of atactic poly(hydroxybutyrate) with a content of unreacted monomers not exceeding 2 % wt. The lactide / glicolide copolymer has a average molecular mass in the range of 190 000 - 300 000, glass transition temperature of 53 - 60°C, melting temperature of 152 - 155°C and enthalpy of fusion above 41 J/g.

The lactide/glycolide copolymer is mass- synthesized with addition of a zircon initiator. The copolymer reveals a chain microstructure that contain both long lactidyl and glycolidyl segments and short alternately arranged elastic sequences of lactide and glycolide

The atactic poly(hydroxybutyrate) is prepared by anionic polymerization of the cyclic β-butyrylolacton in the presence of t-butylamonium acetate

Products according to the invention can also take the form of nonwoven, knitwear and braided band.

The method to produce biodegradable textiles, according to the invention, consists in that the dried up and unpurified blend of the lactide/glycolide copolymer having average molecular mass 190 000 - 300 000, glass transition temperature 53 - 60 ° C, a melting point of 152- 155 ° C and a heat of fusion above 41J / g with a content of unreacted monomers in the amount not exceeding 2% wt, with atactic poly(hydroxybutyrate) and with water content not exceeding 50 ppm is subjected to melting, and the molten polymer mixture is next extruded through a spinneret and formed into textile materials in air atmosphere whereupon the materials are purified and possibly dried

The molten polymer mixture is, according to the invention, extruded at the temperature of 200 - 230°C through a single-or multi-hole spinneret at a speed in the range of 80 - 800 m/min and throughput of 0,3 - 1,9 g/min per one hole. The formed fibers are then drawn at 50 - 60°C with a draw-ratio of 2-4, possibly stabilized at 100°C and up-taken on spools. The thus formed continuous fibers can be cut to produce biodegradable staple fibers.

According to the invention, nonwoven can also be formed by pressing the molten polymer mixture through consequential heating zones at the temperature of 90 - 220°C to a spinning head having the same temperature as the last heating zone. Hot air at 160 - 240°C is blown to the spinning head at the rate of 10 - 40 Nm³/h. The air splits the formed polymer streams to produce the nonwoven which is collected on a take-up device in the distance of 0,1 - 1m from the head. According to the invention, nonwoven can also be formed by pressing the molten polymer mixture through consequential heating zones at the temperature of 90 - 230°C to a spinning head having the same temperature as the last heating zone and collecting the fibres in nonwoven form on a flat electrode under voltage of 10-50 kV in a distance of 1- 10 cm from the head.

The nonwoven can be formed by pressing the molten polymer mixture through consequential heating zones at the temperature of 90 - 230°C and next through a multi- hole spinneret having the same temperature as the last heating zone with throughput of 0.1 - 3.0 g/min/hole. The obtained fiber is drawn by means of air at speed of 800 - 4000 m/min, whereupon a fleece is formed which is integrated on a calendar at temperature of 20 - 50 °C and pressure of 0.1 - 1.0 MPa. The formed nonwoven is collected on a take-up device.

In the invention the method for preparing biodegradable textile materials from a melt may rely on that the dried up and unpurified mixture of the lactide - glycolide copolymer with atactic poly(hydroxybutyrate) possibly with an bioactive nano-additive in the amount not exceeding 10 %, and with water content not exceeding 50 ppm is subjected to melting, and the molten polymer mixture is next extruded through a spinneret and formed into textile materials in air atmosphere whereupon the materials are purified and possibly dried.

Thanks to absence of cytotoxicity, the nonwoven, knitwear and braids may find utility in medical devices like dressings , surgery sutures and bone implants.

Cytotoxicity of the fibers and products thereof was assessed according to standard PN-EN ISO 10993-5:2001 by the method of direct contact on cell line L929 - line of fibroblast cells obtained from subcutaneous fat tissue of mice C3H (ATCC CCL 1) and by the method of indirect contact with the use of polar and non-polar extracts. The culture fluid Eagle without calf serum with addition of 100 j/ml penicillin, 100 µg/ml streptomycin and 2 mM/ml L-glutamine was used as polar extract, while, as non-polar extract, served the culture fluid Eagle with addition of 2% of calf serum, 100 j/ml penicillin, 100 µg/ml streptomycin and 2 mM/ml L-glutamine.

The invention is illustrated with following examples not limiting the scope of the invention.

### Example 1

A polymeric mixture [Blenda PLAGA(90% wt) + a-PHB (10% wt.)], was first prepared in a blending extruder at temperature of 145 - 160°C, characterized by number average molecular mass Mn = 100 000, glass transition temperature Tg = 45 °C, melting temperature Tm = 152 °C, enthalpy of fusion dH = 35 J/g. The mixture contained 90% of the PLAGA copolymer and 10% atactic poly(hydroxybutyrate) (a-PHB). The copolymer PLAGA synthesis was conducted with presence of zircon (IV) acetylacetonate as initiator and proceeded for 12 hours at temperature of 140 - 170°C and next for 24 hours at 100 - 120°C.The obtained copolymer containing 84% of lactidyl mole units and 15% of glycolidyl mole units, unreacted L-lactide below 1 %, was characterized by Mn = 130 000, Tg = 54 °C, Tm = 155 °C and dH = 45 J/g. The atactic poly(hydroxybutyrate) (a-PHB) (Mn = 60 000) was prepared by anionic polymerization of the cyclic β-butyrylolactone in the presence of t - butyloamonium acetate.

The polymeric mixture with water content below 50 ppm was extruded through a single-hole spinneret at 215°C, with throughput of 0,3 g/min, The monofilament was air-cooled, collected on a spool with 82 m/min of speed and then drawn by 4,7 fold in a water bath at 51°C. A monofilament was obtained with structure and composition like of the [Blenda PLAGA + PHB (10% wt.)], with linear density of 7,9 dtex, tenacity of 25,7 cN/tex and elongation of 68 %.

### Example 2

From the polymer blend [Blenda PLAGA (91%wt) + a-PHB (9 % wt.)], with Mn = 100 000, Mw = 230 000, Tg = 46°C, Tm = 150°C, dH = 30 J/g, containing 91% wt of the PLAGA copolymer, characterized by Mn = 130000, Mw = 200 000, Tg = 54°C, Tm = 153°C, dH = 49 J/g, and 9% wt of a-PHB a granulate was obtained. PLAGA copolymer containing 15% of glycolide and a-PHB were prepared as in Example 1. Fibers were spun from granulate with water content of 30 ppm in a process consisting of melt-spinning and drawing. Spinning was carried out on an extruder spinning bank at 207°C through a 12-hole spinneret with speed of 800 m/min and output of 18,6g/min. The fiber was next drawn on a drawtwister on a hot godet at 63 °C and collected on a spool with 496 m/min of speed. Continuous fiber was obtained with a composition and structure like that of [Blenda PLAGA + PHB (9 % wt)] with linear density of 63,3 dtex, tenacity of 24,2 cN/tex, elongation of 21,1%, shrinkage of 14,8 % in water at 37°C. The fiber was subjected to cytotoxicity testing according to standard PN-EN ISO 10993-5:2001. The result was 0 cytotoxicity

### Example 3

From fibers made as in Example 2, a braided band designed for surgery sutures was prepared composed of 12 loosely twisted filaments having a chain microstructure. The yarn, wound on spindle bobbins of the braiding machine, was fed to the interlacing zone with tension of 18 - 22 cN. A compensation of the yarns was secured in the course of braiding on the break-preventing level of 60 - 65 cN. The yarn was prepared as a circular sliver from 8 single wefts with the interlacement of 1:1. The interlacement density was controlled by the take-up speed to the length of 0,5 ± 0,1 / mm. A band was obtained with linear density of 46,7 ± 0,3 tex and thickness of 0,39 ± 0,02 mm. The yarn wound on perforated bobbins was finished by a 5-minutes wash in distilled water at 40°C followed by several immersions in a 95 % refined ethanol. The yarn still on bobbins was dried at ambient temperature of 25°C. Sutures were obtained with tenacity of 19,2 cN/tex and 16,8 cN/tex , conditioned and wet accordingly, and elasticity degree of 64,1%. The yarns made up to pieces of desired length, were wound under sterile conditions onto specially prepared elements of the dressing structure. The yarns designed for medical application were X-ray sterilized with the dose of 28 kGy. The sutures were subjected to cytotoxicity testing according to standard PN-EN ISO 10993-5:2001. The result was 0 cytotoxicity.

### Comparative example

Prior to the forming of fibers, a regranulate was prepared by blending of a mixture of the copolymer of glycolide and L-lactide (PLAGA) with the atactic poly(hydroxybutyrate) [Blenda PLAGA (90% wt) + a-PHB (10% wt.)] having the same properties as in Example 1 with poly(ethylene glycol) PGE 400 (mole mass 400 g/mole) in the amount of 1,5 % calculated on the mass of the polymer mixture. The blending was done in the twin-screw extruder Minilab at 170°C and at 100 rpm of the screw. The extruded monofilament was cut into 2 mm pieces. Such prepared regranulate was extruded through a concurrent twin-screw extruder. Temperature in the singular heating zones of the extruder was in the range from 110°C to 170°C; the screw was rotating at 100 rpm. The polymer melt was pressed with a metering pump at 10 rpm to a spinning head at 180°C. Hot air at 190°C was also blown to the spinning head with the throughput of 20 m³/h, splitting the formed polymer streams, and producing a web. The nonwoven was collected on a take-up device in a distance of 20 cm. The material was next purified by removing the unreacted lactide and other post-reaction residues. A nonwoven 1,5 mm thick was obtained with the surface density of 140 g/m² and free water sorption after 8 hours amounting to 3,5 g _{fluid}/g sample (according to standard EN13726-1:2005)

## Claims

1. Biodegradable textile materials **characterized in that** they contain a plurality of fibers prepared from a melt of a homogeneous polymer mixture having average molecular mass in the range of 100 000 - 270 000, glass transition temperature of 45 - 47°C, melting temperature of 149- 152°C and enthalpy of fusion above 30J/g, consisting in 82-98% wt. of a copolymer of lactide and glycolide having average molecular mass 190 000 - 300 000, glass transition temperature 53 - 60 ° C, a melting point of 152- 155 ° C and a heat of fusion above 41J / g, 2-18% wt. of atactic poly(hydroxybutyrate) and of unreacted monomers in the amount not exceeding 2 % wt.

2. Biodegradable textile materials according to Claim 1 **characterized in that** they are provided as product in nonwoven form.

3. Biodegradable textile materials according to Claim 1 **characterized in that** they are provided as product in knitwear form.

4. Biodegradable textile materials according to Claim 1 **characterized in that** they are provided as product in the form of braided band.

5. Biodegradable textile materials according to Claim 1 **characterized in that** the copolymer of lactide and glycolide has a chain microstructure containing long lactidyle and glycolidyle segments and alternate elastic lactyl and glycolyl sequences, and was obtained by synthesis in the mass in the presence of a zirconium initiator.

6. Biodegradable textile materials according to Claim 1 **characterized in that** the atactic poly(hydroybutyrate) is prepared by an anionic polymerization of the cyclic β-butyryllacton in the presence of t-butylammonium acetate.

7. A method for preparing biodegradable textile materials from a melt **characterized in that** the dried up and unpurified mixture of the lactide - glycolide copolymer with atactic poly(hydroxybutyrate) according to claim 1 and with water content not exceeding 50 ppm is subjected to melting, and the molten polymer mixture is next extruded through a spinneret and formed into textile materials in air atmosphere whereupon the materials are purified and possibly dried.

8. A method according to Claim 7 **characterized in that** the continuous fibers are formed by pressing of the molten polymer mixture at temperature of 200 - 230°C through a spinneret at fiber speed of 80 - 800 m/min with throughput per one hole of 0,3 - 1,9 g/min , and, next, the fibers are drawn at temperature of 50 - 60°C and draw ratio of 2 to 4, possibly stabilized at temperature of about 100 ⁰C and wound up on spools.

9. A method according to Claim 8 **characterized in that** the continuous fibers are subjected to crimping and cutting.

10. A method according to Claim 7 **characterized in that** a nonwoven is formed by pressing the molten polymer mixture through consecutive heating zones at temperature of 90 - 220°C to a spinning head at temperature equal to that of the last heating zone and, at the same time, blowing of air at temperature of 160 - 240°C to the spinning head with throughput of 10 - 40 Nm³/h of dry air which splits the formed polymer streams, and the obtained fiber is collected on a take-up device in a distance of 0.1 to 1m from the spinning head.

11. A method according to Claim 7 **characterized in that** the molten polymer mixture is pressed through the consecutive heating zones at temperature of 90 - 200°C to a forming head at temperature of the last heating zone, and is formed into a nonwoven on a flat electrode under the voltage of 10- 50 kV in a distance of 1 - 10 cm from the head.

12. A method according to Claim 7 **characterized in that** the molten polymer mixture is formed to a nonwoven by pressing it through the consecutive heating zones at temperature of 90 - 230°C and next through a multi-hole spinneret at temperature of the last heating zone with throughput of 0,1 - 3,0 g/min/hole, and the obtained fiber is drawn by means of air at speed of 800 - 4000 m/min, and a fleece is formed which is condensed on a calender at temperature of 20 - 50°C and pressure of 0,1 - 1,0 MPa, and the produced nonwoven is collected on a take-up unit.

## Patentansprüche

1. Biologisch abbaubare Faserstoffe kennzeichnen sich dadurch, dass sie zahlreiche Fasern enthalten, die aus einer Legierung der homogenen Polymermischung von der gewichtsmittleren Molmasse 100000 - 270000, der Glasübergangstemperatur 45 - 47°C, der Schmelztemperatur 149 - 152°C und der Schmelzwärme über 30J/g angefertigt werden, bestehend aus 82-98 Gewichtsprozenten von Copolymer von Lactid mit Glycolid von der gewichtsmittleren Molmasse 190000 - 300000, der Glasübergangstemperatur 53 - 60°C, der Schmelztemperatur 152- 155°C und der Schmelzwärme über 41J/g, Gewichtsprozenten 2-18 von ataktischem Polyhydroxybutyrat sowie aus nicht umgesetzten Monomeren in einer Menge von nicht mehr als 2 Gewichtsprozenten.

2. Biologisch abbaubare Faserstoffe kennzeichnen sich nach Patentanspruch 1 dadurch, dass sie als ein Produkt in Form eines Vliesstoffes auftreten.

3. Biologisch abbaubare Faserstoffe kennzeichnen sich nach Patentanspruch 1 dadurch, dass sie als ein Produkt in Form eines Gewirkes auftreten.

4. Biologisch abbaubare Faserstoffe kennzeichnen sich nach Patentanspruch 1 dadurch, dass sie als ein Produkt in Form eines geflechten Bandes auftreten.

5. Biologisch abbaubare Faserstoffe kennzeichnen sich nach Patentanspruch 1 dadurch, dass der Copolymer von Lactid eine Mikrostruktur der Kette aufweist, die lange Segmente von Lactiden und Glycoliden sowie abwechselnd elastische Sequenzen von Glycolyl und Lactylate enthält und durch eine in der Masse geführten Synthese mit der Teilnahme eines Zirkonium Initiators hergestellt wird.

6. Biologisch abbaubare Faserstoffe kennzeichnen sich nach Patentanspruch 1 dadurch, dass das ataktische Polyhydroxybutyrat durch eine anionische Polymerisation des zyklischen β-Butyrolactons in Anwesenheit von t-Butylammoniumacetat hergestellt wird.

7. Das Verfahren zur Herstellung der biologisch abbaubaren Faserstoffe mit einem Schmelzverfahren kennzeichnet sich dadurch, dass eine getrocknete und ungereinigte Mischung des Copolymers von Lactid - Glycolid mit dem ataktischen Polyhydroxybutyrat nach Patentanspruch 1 mit dem Wassergehalt von nicht mehr als 50 ppm einem Schmelzverfahren unterzogen wird und dann die geschmolzene Polymermischung durch eine Spinndüse gepresst wird und aus ihr Faserstoffe in der Luftatmosphäre geformt, gereinigt und eventuell getrocknet werden.

8. Das Verfahren nach Patentanspruch 7 kennzeichnet sich dadurch, dass die geschmolzene Polymermischung bei der Temperatur von 200 - 230°C als Filamenten geformt und durch eine Spinndüse mit der Fasergeschwindigkeit von 80 - 800 m/Min mit der Leistung von 0,3 - 1,9 g/Min pro eine Öffnung gepresst wird, dann werden die Fasern 2-4 fach bei der Temperatur von 50 - 60°C gedehnt, danach werden die Fasern eventuell, bei der Temperatur von etwa 100°C stabilisiert und auf eine Spule eingewickelt.

9. Das Verfahren nach Patentanspruch 8 kennzeichnet sich dadurch, dass die Filamenten gekerbt und geschnitten werden.

10. Das Verfahren nach Patentanspruch 7 kennzeichnet sich dadurch, dass die geschmolzene Polymermischung in Form eines Vlieses geformt wird, indem sie durch laufende Heizabschnitte bei der Temperatur von 90 - 220°C in einen Formkopf bei der Temperatur des letzten Heizabschnittes gepresst wird bei gleichzeitiger Zufuhr der Luft in den Formkopf bei der Temperatur von 160 -240°C, bei der Ausgabe der trockenen Luft von 10 - 40 Nm³/h, mit Hilfe welcher die geformten Polymerplatten zerschlagen werden, und das geformte Vlies an einem Abnahmegerät in der Entfernung von 0,1 - Im vom Formkopf abgenommen wird.

11. Das Verfahren nach Patentanspruch 7 kennzeichnet sich dadurch, dass die geschmolzene Polymermischung durch laufende Heizabschnitte bei der Temperatur von 90 - 230°C in einen Formkopf bei der Temperatur des letzten Heizabschnittes gepresst und als Vlies auf einer flachen Elektrode unter der Spannung von 10 -50 kV geformt wird, die in der Entfernung von 1 - 10 cm vom Formkopf angebracht wird.

12. Das Verfahren nach Patentanspruch 7 kennzeichnet sich dadurch, dass die geschmolzene Polymermasse als Vlies geformt wird, indem sie durch laufende Heizabschnitte bei der Temperatur von 90 - 230°C und dann durch eine Spinndüse mit vielen Öffnungen bei der Temperatur des letzten Heizabschnittes mit der Leistung von 0,1 - 3,0 g/Min/Öffnung gepresst wird, und erhaltenes Vlies wird mit Hilfe der Luft mit der Geschwindigkeit von 800 - 4000 m/Min gedehnt, wobei daraus ein Vlies geformt wird, das in einem Kalander bei der Temperatur von 20 - 50°C unter dem Druck von 0,1 - 1,0 MPa verbunden wird und das hergestellte Vlies wird an einem Abnahmegerät abgenommen.

## Revendications

1. Matériels fibreux biodégradables, qui se caractérisent par le fait qu'ils contiennent des nombreuses fibres produites d'alliage de mélange polymère homogène dont le poids moyenne molaire est 100000 - 270000, la température de transition vitreuse est 45 - 47°C, la température de point de fusion est 149 - 152°C, et la chaleur de fusion est supérieure de 30J/g, composé de 82-98% du poids de copolymère de lactide avec glicolide du poids moyenne molaire 190000 - 300000, de la température de vitrification est 53 - 60°C, la température de fusion est 152 - 155°C et la chaleur de fusion au-dessus de 41J/g, de 2-18% du poids atactique de poly (hydroxybutyrate) et des monomères que n'ont pas réagi dans la quantité pas plus grande que 2% du poids.

2. Matériels fibreux biodégradables selon la réservation 1, qui se caractérisent par le fait d'apparaître comme les produits sous la forme de non-tissés.

3. Matériels fibreux biodégradables selon la réservation 1, qui se caractérisent par le fait d'apparaître comme le produit sous la forme du tricot.

4. Matériels fibreux biodégradables selon la réservation 1, qui se caractérisent par le fait d'apparaître comme le produit sous la forme de ruban de la tresse.

5. Matériels fibreux biodégradables selon la réservation 1, qui se caractérisent par le fait que le copolymère de lactide avec glicolide possède la microstructure de la chaîne contenant des longs segments de lactide et de glicolide et les séquences élastiques de glicolide et lactide, il est obtenu par le processus de synthèse réalisé dans la masse avec participation d'initiateur de zirconium.

6. Matériels fibreux biodégradables selon la réservation 1, qui se caractérisent par le fait que poly (hydroxybutyrate) atactique est obtenu par le processus de polymérisation anionique de β-butyrolactone cyclique en présence d'acétate de t-butyloammonium.

7. La manière de fabrication des matériels fibreux biodégradables par la méthode d'alliage, qui se caractérisent par le fait que le mélange, séché et non nettoyé, de copolymère lactide - glicolide avec poly (hydroxybutyrate) atactique selon la réservation 1 avec le contenu d'eau non supérieur que 50 ppm, il est soumis à la fusion, et ensuite le mélange fondu de polymère est pressé par de la filière et on forme d'elle les matériaux fibreux dans l'atmosphère d'air, ils sont purifiés et séchés éventuellement.

8. La manière selon la réservation 7 qui se caractérisent par le fait que, le mélange fondu de polymère avec la température 200 - 230°C se forme dans l'aspect des fibres continues en les pressant par la filière avec la vitesse de fibre 80 - 800 m/min avec la capacité de 0,3 - 1,9 g/min par l'oeillet, ensuite les fibres sont étendues avec la multiplication de 2 - 4 dans la température de 50 - 60°C, et après éventuellement on les stabilise dans la température d'environ 100°C et on les enroule sur les bobines.

9. La méthode selon la réservation 8 se **caractérise par** cela, que les fibres sont soumises de l'ondulation et du coupage.

10. La méthode selon la réservation 8 se **caractérise par** cela que le mélange des polymères est formé en aspect de non-tissé en les pressants par les sections suivantes de chauffage avec la température de 90 - 220°C à la tête de forme de la température de la dernière section de chauffage, avec la conduction en même temps de l'air avec la température de 160 -240°C, avec le débit de l'air sec de 10 - 40 Nm³/h, lequel brise les flots formés de polymères, et le non-tissé obtenu est ramassé sur l'appareil de réception à l'espace de 0,1 - lm de la tête.

11. La méthode selon la réservation 7 se **caractérise par** cela que le mélange des polymères est pressé par les sections suivantes de chauffage avec la température de 90 - 220°C à la tête de forme de la température de la dernière section de chauffage et il est formé en aspect de non-tissés sur l'électrode plat sous la tension de 10 -50 kV, placé à la distance de - 10 cm de la tête.

12. La méthode selon la réservation 8 se **caractérise par** cela que la masse de polymères est formée en aspect de non-tissé en la pressant par les sections suivantes de chauffage dans la température de 90 - 230°C et ensuite par la filière à multi-trous avec la température de la dernière section de chauffage, avec la capacité de 0,1 - 3,0 g/min/trou, et les fibres obtenues sont étendues à l'aide de l'air avec la vitesse de 800 - 4000 m/min, et ensuite on forme la toison qui est uni sur la calandre dans la température de 20 - 50°C sous la tension de 0,1 - 1,0 MPa et non-tissé formé est repris sur l'appareil de réception.
